# FASCICULE DE BREVET EUROPEEN

(11) **EP 1 789 802 B1**
(45) Date de publication et mention de la délivrance du brevet: **03.06.2009**
(21) Numéro de dépôt: 05797071.7
(22) Date de dépôt: 15.09.2005
(51) Int. Cl.: G01N 33/574, G01N 33/50

(54) **VIMENTINE PHOSPHORYLEE COMME MARQUEUR DE L'AGRESSIVITE ET/OU L'INVASIVITE DES TUMEURS**
PHOSPHORYLIERTE VIMENTIN ALS MARKER FÜR DIE AGGRESSIVITÄT UND /ODER INVASIVITÄT VON TUMOREN
PHOSPHORYLATED VIMENTIN SERVING AS A MARKER OF THE AGGRESSIVENESS AND/OR INVASIVENESS OF TUMORS

(30) Priorité: 17.09.2004 FR 0409857
(43) Date de publication de la demande: 30.05.2007
(73) Titulaire: INSTITUT NATIONAL DE LA SANTE ET DE LA RECHERCHE MEDICALE (INSERM), 75013 Paris (FR)
(72) Inventeur: BOUAMRANI, Ali, F-38100 GRENOBLE (FR); GAY, Emmanuel, F-38240 MEYLAN (FR); ISSARTEL, Jean-Paul, F-38120 SAINT EGREVE (FR); BERGER, François, F-38240 MEYLAN (FR)
(74) Mandataire: Tetaz, Franck Claude Edouard
(86) Numéro de dépôt international: PCT/EP2005/054598
(87) Numéro de publication internationale: WO 2006/030023

(56) Documents cités:
- EP-A- 1 067 142
- WO-A-03/073821
- WO-A-03/076651
- MOR-VAKNIN N ET AL: "Vimentin is secreted by activated macrophages" NATURE CELL BIOLOGY, MACMILLAN PUBLISHERS, GB, vol. 5, janvier 2003 (2003-01), pages 59-63, XP002978115 ISSN: 1465-7392 cité dans la demande
- NAKAMURA YU ET AL: "Localized phosphorylation of vimentin by Rho-kinase in neuroblastoma N2a cells" GENES TO CELLS, vol. 5, no. 10, octobre 2000 (2000-10), pages 823-837, XP002333988 ISSN: 1356-9597
- KOPEREK OSKAR ET AL: "Value and limits of immunohistochemistry in differential diagnosis of clear cell primary brain tumors" ACTA NEUROPATHOLOGICA, vol. 108, no. 1, juillet 2004 (2004-07), pages 24-30, XP002333989 ISSN: 0001-6322
- LIM YOON-PIN ET AL: "Selective tyrosine hyperphosphorylation of cytoskeletal and stress proteins in primary human breast cancers: implications for adjuvant use of kinase-inhibitory drugs." CLINICAL CANCER RESEARCH : AN OFFICIAL JOURNAL OF THE AMERICAN ASSOCIATION FOR CANCER RESEARCH. 15 JUN 2004, vol. 10, no. 12 Pt 1, 15 juin 2004 (2004-06-15), pages 3980-3987, XP002333990 ISSN: 1078-0432
- TING A Y ET AL: "Genetically encoded fluorescent reporters of protein tyrosine kinase activities in living cells" PROCEEDINGS OF THE NATIONAL ACADEMY OF SCIENCES OF USA, NATIONAL ACADEMY OF SCIENCE. WASHINGTON, US, vol. 98, no. 26, 18 décembre 2001 (2001-12-18), pages 15003-15008, XP002903907 ISSN: 0027-8424

## Description

La présente invention concerne un nouveau procédé susceptible d'être employé pour déterminer l'agressivité et/ou l'invasivité d'une tumeur ainsi qu'un procédé pour identifier un agent anticancéreux permettant de réduire l'agressivité et/ou l'invasivité d'une tumeur.

L'invention est basée sur la constatation par les inventeurs que la vimentine produite par les cellules cancéreuses pouvait être un marqueur de l'agressivité et/ou de l'invasivité d'une tumeur.

Cette qualité de marqueur n'était pas liée au niveau d'expression de la vimentine dans les cellules cancéreuses, mais sur une transformation post-traductionnelle de cette protéine différente selon le degré d'agressivité et/ou d'invasivité.

La vimentine phosphorylée, est détectable spécifiquement dans les tumeurs non-invasives. A l'inverse, ce marqueur est en quantité significativement beaucoup plus faible dans des tumeurs de mauvais pronostic, à savoir des tumeurs dont le caractère invasif met en périt la survie des patients présentant ce genre de tumeurs.

MOR-VAKNIN N ET AL: "Vimentin is secreted by activated macrophages" NATURE CELL BIOLOGY, MACMILLAN PUBLISHERS, GB, vol. 5, janvier 2003, pages 59-63, décrit la sécrétion de vimentine par les macrophages. La phosphorylation de la vimentine augmente sa sécrétion dans le milieu extracellulaire. La vimentine serait secrétée par les macrophages en réponse a un signal pro-inflammatoire et serait impliquée dans les fonctions immunes.

NAKAMURA YU ET AL: "Localized phosphorylation of vimentin by Rho-kinase in neuroblastoma N2a cells" GENES TO CELLS, vol. 5, no. 10, octobre 2000, pages 823-837, décrit une étude de la distribution de vimentine phosphorylée dans des cultures cellulaires de neuroblastomes.

KOPEREK OSKAR ET AL: "Value and limits of immunohistochemistry in differential diagnosis of clear cell primary brain tumors" ACTA NEUROPATHOLOGICA, vol. 108, no.1, juillet 2004, pages 24-30, décrit l'analyse de tumeurs du cerveau par immuno-histochimie. Des anticorps anti-vimentine (anti-VIM) permettent de différencier les tumeurs.

WO 03/076651 décrit un procédé pour identifier un agent anticancéreux dans lequel on met en contact de cellules produisant la protéine Araf1 kinase avec l'agent à tester. Les agents identifiés sont utilisés pour la préparation d'un médicament pour le traitement du cancer.

Le diagnostic neuropathologique des tumeurs combine l'évaluation de deux paramètres fondamentaux (Chatel et Brucher, 2001 ; Daumas-Duport et Figarella-Branger, 2002):
- Un critère cytologique, qui correspond au typage cellulaire. Ce typage consiste à déterminer l'origine histologique de la tumeur. On distingue, par exemple, des astrocytomes (tumeurs des cellules astrocytaires), des oligodendrogliomes (tumeurs des oligodendrocytes), des méningiomes (tumeurs des méninges) ; ainsi que de nombreux autres types de tumeurs
- Un critère d'histopronostique, qui en fonction d'éléments d'observations microscopiques de la morphologie des cellules, consiste à attribuer à la tumeur un grade de malignité. On peut catégoriser les tumeurs selon une échelle de quatre grades maximum (selon les classifications en vigueur utilisées) qui prédisent le degré d'évolution et d'invasivité de la lésion tumorale. Certaines tumeurs sont peu évolutives, d'autres croissent très rapidement.

A l'heure actuelle, cette analyse histologique réalisée par l'anatomo-pathologiste est prédominante, malheureusement les discordances diagnostiques observées entre experts du domaine sont énormes (jusqu'à 64% de désaccord selon les tumeurs). Pire, des discordances similaires peuvent être notées lorsque les interprétations d'échantillons identiques sont confiées à la même personne à quelques semaines d'intervalles. Ce constat est inquiétant quand on sait que des erreurs diagnostiques peuvent entraîner une radiothérapie et/ou une chimiothérapie inutile et lourde de conséquences pour le patient.

En complément de l'analyse histologique, il n'existe que quelques rares tests-diagnostics basés sur des approches moléculaires. Les observations cytologiques peuvent être ainsi complétées par la recherche d'anomalies génétiques et dans quelques laboratoires de la détection de certains marqueurs protéiques à l'aide d'anticorps spécifiques.

On peut citer à titre d'exemples :
- pour les analyses cytogénétiques : la recherche d'anomalies chromosomiques telles que délétions, amplifications de régions, pertes d'hétérozygoties (notamment pertes alléliques en 1p et 19q pour les oligodendrogliomes (Cairncross et al, 1998)), recherches de marqueurs signant les fragmentations et translocations de chromosomes. Ces événements qui peuvent affecter diverses régions chromosomiques de plusieurs chromosomes sont souvent détectés à l'aide de méthodes telles que : définition du caryotype, Hybridation Génomique Comparative (CGH), hybridation de sondes fluorescentes *in situ* (FISH) ou par des techniques basées sur la PCR.
- pour la recherche de marqueurs protéiques : la détection immunohistochimique de la protéine gliofibrillaire acide (GFAP) indicatrice de l'astrocyte normal ou tumoral ; de l'antigène de prolifération Ki 67.

Il n'existe pas en routine, pour l'instant, de tests basés sur une détection et quantification de concentration de marqueurs transcriptomiques des tumeurs.

Les quelques tests moléculaires disponibles actuellement ne permettent pas de distinguer sans ambiguïté les différents types de cellules tumorales et surtout, de pronostiquer correctement leur agressivité et invasivité.

La pertinence du diagnostic a une incidence prépondérante sur l'action thérapeutique engagée par le clinicien qui dispose d'une panoplie de moyens de lutte contre les tumeurs.

L'exérèse de la tumeur constitue un moyen, a priori radical, pour éradiquer une tumeur. Dans la pratique clinique cette stratégie reste imparfaite. L'ablation de la tumeur se doit d'être exhaustive, ce qui est loin d'être le cas soit parce que les limites de la tumeur restent difficiles à évaluer lors du diagnostic, soit que les tumeurs au caractère infiltrant ont déjà « irradié» des cellules pré-tumorales dans les régions cérébrales voisines à la tumeur primitive. Enfin, la chirurgie constituant un acte traumatisant à la base, l'opérabilité des tumeurs doit toujours être évaluée de manière critique en fonction du bilan bénéfice-risque pour le patient.

Il est donc important de pouvoir disposer de nouvelles méthodes permettant de caractériser l'agressivité et/ou l'invasivité d'une tumeur.

La vimentine est une protéine de 465 acides aminés de masse moléculaire 53554 Da (Swiss-Prot (http://www.expasy.org) numéro d'accession: P08670).

Le gène codant la vimentine est localisé sur le chromosome 10 dans la région 10p13 (Ferrari et al, 1987). Il existe une copie unique de ce gène dans le génome haploïde humain (un pseudogène a été identifié sur le chromosome 6 ; région cytogénétique 6q22.32). Un transcrit unique d'environ 1,8kbp est habituellement détecté dans les cellules exprimant la vimentine (Lilienbaum et al, 1986 ; Perreau et al, 1988). Plusieurs polymorphismes ou conflits de séquençage sont reportés dans la littérature (Ferrari et al, 1986 ; Perreau et al, 1988 ; Honoré et al, 1990).

Selon des explorations immunohistochimiques, la vimentine, tout comme la GFAP, ne seraient retrouvées ni dans les médulloblastomes, ni dans les oligodendrogliomes (Yung et al, 1985), mais bien présentes dans tous les astrocytomes (Yang et al, 1994).

De nombreuses données dans la littérature suggèrent que dans les cellules cancéreuses épithéliales il y a expression aberrante de vimentine. Ce phénomène d'expression de protéines des filaments intermédiaires dans les cellules épithéliales, avec acquisition de propriétés migratoires et/ou invasives est appelé transition épithélio-mésenchymale (EMT).

La vimentine est détectée dans de nombreux carcinomes (tumeurs épithéliales) mammaires hormone-indépendants (Cattoretti et al, 1988 ; Sommers et al, 1989). L'hyperexpression de vimentine serait à mettre en corrélation avec un caractère invasif et métastasique marqué dans le cas du carcinome du col de l'utérus (Gilles et al, 1996). La détection de vimentine dans les cellules de carcinomes de rein est un marqueur de mauvais pronostic (Donhuijsen et Schulz, 1989) ; dans ces tumeurs, il y aurait relation entre expression de vimentine, contenu en ADN nucléaire et grade histologique (Dierick et al, 1991). Pour certaines lignées cellulaires tumorales de la prostate, la motilité mais pas l'invasivité peut être mise en corrélation avec une plus forte expression de la vimentine *in vitro.* Ces tumeurs qui expriment la vimentine sont susceptibles de produire des métastases osseuses (Lang et al, 2002). Les cellules d'hépatocarcinome Hep3B traitées par les esters de phorbol (activateurs de Protéines Kinases C, PKC) ou par l'acide rétinoïque montrent respectivement une augmentation du taux de transcrit de la vimentine ou une diminution. Ces composés n'affectent pas la prolifération cellulaire mais le potentiel invasif, mesuré *in vitro,* est augmenté par les esters de phorbol et réduit avec l'acide rétinoïque (Yoon et al, 2004). En outre, la coexpression de vimentine et de kératines dans les cellules tumorales de mélanomes ou de cancers mammaires est jugée par plusieurs auteurs comme un facteur d'accroissement des capacités prolifératives et métastasiques de ces tumeurs (Chu et al, 1996, Hendrix et al, 1997).

Selon les publications mentionnées ci-dessus, il apparaît crédible de conclure que l'invasivité des cellules tumorales serait directement proportionnelle à la concentration cellulaire de vimentine. Néanmoins, on trouve aussi d'autres travaux qui ne confirment pas ce message. Dans les tumeurs mammaires, il a été indiqué que l'expression de la vimentine est un élément qui ne doit pas être associé avec un risque accru de décès et être considéré comme un facteur de mauvais pronostic (Seshadri et al, 1996). Pour d'autres auteurs, la mesure du taux d'expression de la vimentine n'est pas un paramètre qui permet de poser un diagnostic ni de prévoir les capacités d'évolution des tumeurs (Raymond and Leong, 1989 ; Holck et al, 1993 ; Heatley et al, 1995, 2002).

En clair, le niveau d'expression de la vimentine ne corrèle pas nécessairement avec l'invasivité selon les lignées cellulaires testées. Le contrôle transcriptionnel de l'expression de la vimentine n'apparaît donc pas comme le seul critère permettant de rationaliser l'invasivité des tumeurs.

La vimentine peut exister sous une forme non-phosphorylée et de multiples formes phosphorylées. La phosphorylation de la vimentine joue un rôle important dans le désassemblage des filaments intermédiaires. Plusieurs résidus de sérines majoritairement situés dans la partie N terminale de la protéine constituent les sites de modification post-traductionnelle par phosphorylation (Ando et al, 1989, 1991 ; Chou et al, 1991 ; Huang et al, 1994). Plusieurs kinases différentes peuvent être impliquées dans ces modifications de la vimentine (Tsujimura et al, 1994). Dans les cellules en cours de prolifération, la vimentine présente différents statuts : phosphorylée ou non-phosphorylée en fonction des différentes étapes des mitoses (Tsujimura et al, 1994).

Par Agressivité, on entend selon l'invention la caractéristique définissant le caractère malin des tumeurs. Les tumeurs sont généralement classifiées selon plusieurs grades (les grades sont établis sur une échelle évoluant, en majorité et en fonction des types de tumeurs, sur des niveaux de 1 à IV). Le grade est défini en combinant des critères divers basés sur l'observation clinique, les paramètres histologiques et cytologiques.

Par Invasivité, on entend selon l'invention la faculté pour une tumeur maligne de haut grade de se propager en dehors du tissu au sein duquel elle a pris naissance. Cette propagation va de pair avec une colonisation de nouvelles structures histologiques, la tumorisation de tissus adjacents et/ou la possibilité d'envahissement plus ou moins généralisable de l'organisme par voies de métastases.

La présente invention concerne donc un procédé susceptible d'être employé pour déterminer l'agressivité et/ou l'invasivité d'une tumeur, ledit procédé comprenant, sur un échantillon de cellules tumorales préalablement prélevé les étapes de :
- préparer un extrait biologique comprenant de la vimentine, et
- déterminer si la vimentine ainsi extraite est phosphorylée ou non phosphorylée, la présence significative de vimentine phosphorylée étant un indice de non-agressivité et/ou non-invasivité de la tumeur.

Pour la méthode selon l'invention, on pourra établir la teneur moyenne en vimentine phosphorylée sur des échantillons de cellules tumorales connues pour leur agressivité et/ou invasivité, puis ensuite comparer la teneur en vimentine phosphorylée d'un nouvel échantillon à analyser avec cette teneur moyenne, valeur standard d'invasivité et/ou d'agressivité.

C'est par rapport à la teneur moyenne associée aux tumeurs invasives que l'on pourra déterminer si la teneur en vimentine phosphorylée de l'échantillon à analyser est significativement importante - indice de non-invasivité et/ou de non-agressivité - ou significativement faible - indice d'invasivité et/ou d'agressivité -.

Pour la méthode selon l'invention, on pourra également établir la teneur moyenne en vimentine phosphorylée sur des échantillons de cellules tumorales connues pour leur non-agressivité et/ou non-invasivité, puis ensuite comparer la teneur en vimentine phosphorylée d'un nouvel échantillon à analyser avec cette teneur moyenne, valeur standard de non-invasivité et/ou de non-agressivité.

C'est par rapport à cette teneur moyenne associée aux tumeurs non invasives et/ou non agressives que l'on pourra déterminer si la teneur en vimentine phosphorylée est significativement importante ou faible.

L'homme du métier pourra choisir d'effectuer une comparaison par rapport à une seule des deux valeurs standard ou par rapport aux deux valeurs standard ci-dessus.

Selon un autre mode de réalisation de l'invention, on pourra déterminer sur le même échantillon la teneur en vimentine phosphorylée et la teneur en vimentine non phosphorylée, la comparaison des deux valeurs permettent de déterminer si la teneur en vimentine phosphorylée est significativement importante ou faible.

Pour la mise en oeuvre du procédé selon l'invention, on détermine si la vimentine est phosphorylée ou non phosphorylée de préférence au moyen d'une méthode sélectionnée parmi le groupe comprenant :
- l'analyse SELDI-TOF
- la méthode FACS ou ses équivalents
- les marquages isotopiques RIA
- le transfert de fluorescence FRET
- la mise en évidence de complexe-immuns
- les méthodes permettant de détecter des réactions antigènes-anticorps comme les méthodes ELISA, les micro-arrays de protéines ou Western-blot
- les tests de migration électrophorétique avec mise en évidence de modifications post-traductionnelles de la vimentine.

Selon le mode de détection choisi, la détection de la vimentine phosphorylée ou non dans l'extrait biologique la comprenant comprend la mise en évidence de la vimentine et/ou des fragments de vimentine.

L'homme du métier saura déterminer les fragments de vimentine permettant de mettre en oeuvre le procédé selon l'invention.

Selon un premier mode de réalisation de l'invention, la vimentine modifiée par phosphorylation est extraite d'un échantillon de nature biopsique puis analysée et détectée par l'approche expérimentale basée sur la spectrométrie de masse dite technologie SELDI (Surface Enhanced Desorption/Ionization technology) (Merchant et Weinberger, 2000 ; Weinberger et al, 2000). Brièvement, cette plate-forme allie un enrichissement en fractions protéiques, à partir d'un échantillon biologique, sur un support de type matrice chimique activée, couplé à une analyse par spectrométrie de masse des protéines ainsi adsorbées sur le support. Ce mode de réalisation est effectuée en exploitant la plateforme technique distribuée par la société Ciphergen (Ciphergen Biosystems, Inc. Fremont, CA, USA ; http://www.ciphergen.com). L'exemple 1 illustre les résultats obtenus pour l'analyse de divers types de tumeurs invasives ou non-invasives (tumeurs des méninges, tumeurs des cellules gliales du système nerveux central (glioblastomes et oligodendrogliomes) et tumeurs du poumon).

Néanmoins, il est également possible de faire appel à d'autres procédés ou méthodes d'analyse pour constituer une application à visée diagnostique ou pronostique de l'état d'invasivité des tumeurs et reposant dans son principe de base sur la détection et/ou le dosage du marqueur identifié, objet de l'invention, ou sur tout produit issu de ce marqueur ainsi que sur les précurseurs de ce marqueur, d'une part, ou les éléments biologiques qui interviennent dans sa synthèse, dégradation ou modification post-traductionnelle, d'autre part. En outre, la mise en place de tests diagnostiques peut nécessiter que soient détectées voire quantifiées la vimentine non modifiée et la vimentine phosphorylée. Ceci peut être requis par exemple dans le but de procéder à des mesures de rapports de quantités entre les formes de vimentine non-phosphorylée et les formes phosphorylées.

Selon un mode de réalisation autre que la méthode SELDI, la vimentine phosphorylée peut être tout d'abord purifiée de l'extrait obtenu à partir des échantillons selon des méthodes de biochimie classiques puis détectée selon des méthodes choisies parmi celles mentionnées ci-dessous à titre d'exemple. Dans ce cas, l'extraction de la protéine phosphorylée ne repose pas, comme dans le cas de la réalisation par la méthode SELDI sur la capture par affinité de la protéine phosphorylée sur des surfaces de matériau activé (type ProteinChip® de Ciphergen). Après enrichissement de l'extrait en vimentine phosphorylée, voire purification de la-dite protéine, la détection de cette protéine peut être obtenue selon l'une des 2 approches décrites ci-après qui permettent la détection de la totalité ou de fragments correspondants à, ou issus de la protéine sous sa forme non-phosphorylée:
1. La vimentine non modifiée peut être détectée comme telle après élimination chimique ou enzymatique des groupements phosphates (déphosphorylation) de la vimentine phosphorylée extraite des échantillons. Cette approche, illustrée dans l'exemple 2 , peut être réalisée en complément du test de détection de la vimentine phosphorylée et permettrait dans ce cas de confirmer que la vimentine phosphorylée est bien présente dans l'échantillon analysé.
2. Enfin, il est également envisageable de détecter la présence ou la disparition des peptides issus d'un clivage enzymatique ou chimique de la vimentine non modifiée. Une illustration de résultats d'analyse obtenus par la détection de peptides de digestion enzymatique est présentée dans l'exemple 3. En outre, le clivage de la vimentine phosphorylée par des réactions chimiques ou enzymatiques incluant des modifications chimiques des acides aminés phosphorylés (selon des méthodes s'inspirant de celles décrites par exemple par Rusnak et al, 2002 ou Knight et al, 2003) pourraient conduire à la création de peptides de vimentine identiques à des fractions de vimentine non modifiée et qui seraient alors détectables comme tels selon des méthodes appropriées.

D'autres techniques d'analyse sont également susceptibles d'être employées pour détecter la ou les formes de vimentine, phosphorylée(s) ou non, pour la réalisation d'un test biologique d'analyse à visée diagnostique. La liste non-exhaustive décrite ci-après à valeur d'exemple de techniques variées que l'homme de l'art est en mesure d'utiliser pour réaliser une analyse d'échantillons de tumeurs afin de détecter tout ou partie de la vimentine modifiée et d'en tirer une information à valeur diagnostique ou pronostique conformément à l'invention décrite ici. Les analyses pourront être réalisées directement sur des prélèvements bruts, fragments ou coupes de tissus (biopsies) ou sur des prélèvements ayant subit des traitements, correspondant de manière non-exhaustive à des lysats, extraits ou sous-fractions issus de ces tumeurs. Il est également envisageable de procéder à la recherche du marqueur ou de ces dérivés dans les liquides biologiques prélevés chez les patients.

Le principe de base général utilisable consiste à disposer de composants (composants que l'on nommera réactifs) susceptibles d'interagir avec la ou les formes de vimentine ou leurs dérivés. Au chapitre de ces réactifs on peut citer bien évidemment, les anticorps polyclonaux ou monoclonaux ainsi que leurs fragments immunoréactifs, greffés ou non sur, ou avec d'autres composants ; des éléments particulaires susceptibles d'interagir avec les formes de vimentine (phages ou bactéries recombinants exprimant à leur surface des régions polypeptidiques capables d'interaction avec des haptènes ou antigènes) (Gao et al, 1999 ; Knappik et al, 2000) ; ou des aptamères (molécules chimiques de type polynucléotides voire polypeptidiques capables d'établir des interactions non covalentes de forte affinité avec des molécules cibles) (Ellington et Szostak, 1990 ; Tuerk et Gold, 1990). Ces réactifs sélectifs autorisent une mise en évidence de la vimentine modifiée de manière extrêmement fiable et selon les approches, une quantification du taux de cette protéine dans l'échantillon.

L'utilisation de tels réactifs sélectifs peut être envisagée au travers de méthodes ou de protocoles variés qui sont évoqués ci-dessous.

### Analyses basées sur les techniques d'immuno-histo-chimie (Kiernan, 1999) :

Une approche robuste et relativement simple dans son principe consiste à détecter dans des coupes, frottis ou autres préparations provenant des biopsies une analyse de type immuno-histologique. Dans ce cas il s'agit donc typiquement de tests réalisés sur un échantillon brut (coupe de tumeurs constituées de cellules plus ou moins homogènes). L'application de cette technique consiste à mettre en évidence une réaction entre les réactifs et la vimentine sur la préparation. La mise en évidence des complexes (protéines détectés-réactifs) impose la détection d'un signal généré par l'utilisation de traceurs radioactifs, de réactifs fluorescents par exemple ou de méthodes colorimétriques. Les cellules tumorales non-invasives contenant de la vimentine phosphorylée montreront une réaction positive avec le réactif sélectionné spécifique vis-à-vis de la vimentine phosphorylée. A l'inverse, les cellules invasives ne contenant pas de quantités détectables de vimentine modifiée ne montreront pas de signal.

Tests basés sur le tri cellulaire utilisant la fluorescence (méthode dite cytométrie de flux ou FACS, Fluorescent Activated Cell Sorting) (Hulett et al, 1969 ; Parks et Herzenberg, 1984) : un réactif marqué par un groupement fluorescent pourra être utilisé afin de marquer des cellules entières issues et isolées de biopsies et de permettre le tri et la quantification des cellules positives pour la présence de vimentine modifiée. Dans le cadre de cette méthode, les cellules non-lysées dissociées du tissu biopsique fraîchement collecté sont mises en contact du réactif sélectif fluorescent et la suspension et ensuite analysée à l'aide d'un trieur de cellule. Le trieur de cellules détecte de manière individuelle l'intensité du signal fluorescent associé à chaque cellule, isole les cellules dans des réservoirs spécifiques et procède au comptage du nombre de cellules ainsi sélectionnées. Des instruments conçus pour l'analyse FACS sont distribués par exemple par la société Becton Dickinson (Franklin Lakes, NJ, USA ; http://www.bd.com). L'utilisation de paramètres techniques appropriés et optimisés (dilution des cellules, paramètres optiques,...) permet d'envisager le tri et comptage sélectif des cellules contenant de la vimentine modifiée ou non et d'assurer la faisabilité de cette méthode d'utilisation de l'invention décrite ici. A noter cependant, qu'une approche de cytométrie de flux appliquée à l'analyse des extraits protéiques (ie, sur les cellules lysées cette fois) pourrait être conduite par exemple avec la technologie Bio-Plex de Bio-Rad (Hercules, CA, USA ; http://www.bio-rad.com).

Les deux méthodes décrites ci-dessus ne requièrent pas nécessairement la préparation d'un extrait de cellules puisqu'elles peuvent être appliquées directement sur des cellules. D'autres méthodes (basées surtout sur des réactifs de type anticorps) listées ci-après nécessitent quant à elles la préparation d'un extrait protéique obtenu après lyse de l'échantillon à analyser. Ces méthodes sont basées sur l'analyse de l'interaction entre le réactif et la protéine à détecter, incluant éventuellement l'adsorption de cette dernière sur un support (méthodes ELISA, RIA, FRET, micro-arrays, détecteurs utilisant la résonance plasmonique de surface...). La génération d'un signal associé à cette interaction autorise la détection de la vimentine modifiée dans un échantillon et sa quantification, en fonction de l'intensité des signaux engendrés. Ainsi, des anticorps dirigés contre la vimentine modifiée peuvent être utilisés pour interagir (voire dans certains cas capturer) sélectivement la protéine d'intérêt dans un mélange complexe constitué d'un extrait contenant la quasi-totalité des protéines contenues dans une biopsie de tumeurs. L'interaction entre l'anticorps et la vimentine modifiée conduit à la formation d'un complexe binaire, dit complexe-immun, composé de vimentine modifiée et d'anticorps. Dans la plupart des cas et comme pour la méthode de détection préférée par les auteurs décrite dans ce document, les méthodes présentées ci-après s'adressent plus particulièrement, mais sans exclusive, à des lysats d'échantillons biopsiques.

Tests basés sur les marquages isotopiques et le RIA (radio-immuno assays) (Yalow et Berson, 1960 ; Booth et al, 1982) : dans une des variantes de ce dosage, le complexe-immun est réalisé, en rajoutant dans le milieu réactionnel, outre l'extrait protéique de biopsie et l'anticorps, une quantité connue de vimentine modifiée marquée par un isotope radioactif. La vimentine modifiée présente dans l'extrait biologique et la vimentine radioactive-traceur vont entrer en compétition pour la fixation sur l'anticorps. Après sélection des complexe-immuns formés, la quantité de radioactivité détectable dans la fraction ainsi isolée est inversement proportionnelle à la quantité de vimentine modifiée présente dans un échantillon biopsique. Des trousses diagnostiques utilisant le principe du RIA sont distribuées par exemple, pour divers dosages, par la société Schering / Cis-Bio International (Gif/Yvette, France ; www.cisbiointernational.fr).

Tests basés sur les méthodes de transfert de fluorescence, FRET (Fluorescence Resonance Energy Transfer) : pour une telle analyse, le dosage peut, dans sa version la plus attractive, être réalisé directement en solution (dosage en phase homogène) et ne nécessite pas d'isoler ou purifier l'un ou l'autre des composants du complexe-immun. Cette méthode nécessite dans une de ces variantes, l'utilisation de deux anticorps différents dirigés contre la vimentine modifiée et marqués par des groupements fluorescents appropriés. Les deux groupements fluorescents sont sélectionnés de telle sorte que leurs caractéristiques optiques permettent pour l'un des groupements d'être excitable par le rayonnement lumineux utilisé pour la mesure de fluorescence, puis autorisent le transfert de l'énergie d'excitation au deuxième groupement fluorescent qui émet, en dernier ressort, un rayonnement de fluorescence de longueur d'onde bien spécifique. Le transfert de fluorescence n'est effectif que si les deux molécules sont maintenues à proximité suffisamment rapprochée l'une de l'autre. De fait, dans ce type de dosage, les deux anticorps marqués par les deux groupements fluorescents sont choisis pour pouvoir se fixer de manière simultanée sur la vimentine modifiée. Le complexe-immun ternaire formé (anticorps fluorescent d'excitation - vimentine - anticorps fluorescent d'émission lumineuse) permet donc un rapprochement de deux anticorps et dans ce cas seulement, un signal de fluorescence peut être détecté. L'intensité du signal de fluorescence mesuré est donc directement proportionnelle à la quantité de vimentine modifiée présente dans l'extrait biologique (Mathis, 1995 ; Szollosi et al, 1998 ; Blomberg et al, 1999 ; Ueda et al, 1999 ; Enomoto et al, 2000). L'analyse de protéines par la méthode de transfert de fluorescence peut être effectué sur l'appareil Krypton® de la société allemande B.R.A.H.M.S. (www.brahms.de).
Tests de détection basés sur la mise en évidence macroscopique des complexe-immuns (test au latex, bandes d'immuno-détection) (Singer et al, 1957 ; Hechemy et al, 1974) : dans ce type de système de détection, des anticorps anti-vimentine modifiée sont couplés chimiquement à des composants particulaires de tailles micrométriques telles que des billes de polymères colorées ou non. L'incubation en milieu liquide d'une suspension fluide de ces billes recouvertes d'anticorps avec l'extrait biologique à analyser conduit à la création de complexe-immuns agrégeant plusieurs molécules de vimentine et plusieurs billes de polymères. Cette agrégation se traduit par la formation de paquets de billes dont la taille devient macroscopiquement importante au point d'être visibles « à l'oeil » par un opérateur. Dans une variante commune et sophistiquée du système, les complexe-immuns sont soumis à migration par capillarité sur une bandelette de support de chromatographie et révélés par création d'une bande colorée indiquant la présence ou l'absence de l'antigène détecté (test symbolisé par les bandelettes d'immunodétection largement utilisées en routine par exemple pour les tests de grossesses). Des tests au latex sont commercialisés par la société Bio-Mérieux pour des diagnostics microbiologiques par exemple (www.biomerieux.com).

Tests impliquant une capture sur support : Différents tests de détection de réactions antigène-anticorps peuvent être pratiqués exploitant divers modes d'interactions ou adsorptions des composants avec des supports, cupules de réactions ou systèmes de microdétection. Ces tests allient très souvent de hauts niveaux de performance, liés aux sensibilités élevées des méthodes, à la relative simplicité de manipulation des échantillons, à la robustesse des tests, et aux capacités de traitement à haut débit de nombreux échantillons en simultané.
a) méthodes dérivées de l'ELISA. Une des méthodes désormais classique, appelée ELISA (Enzyme Linked Immunoassays) (Engvall et Perlmann, 1971, 1972 ; Engvall et al, 1971) consiste à provoquer la création des complexe-immuns sous forme immobilisée sur les parois des puits (cupules) de plaques de dosages multi-puits en matière plastique. Ce type de méthode se décline en une multitude de variantes selon que les protocoles reposent sur l'immobilisation première des anticorps ou des antigènes dans le fond des puits et selon les méthodes de révélation utilisées (ELISA direct ou indirect). A titre d'exemple, on peut brièvement mentionner que ce genre de test peut être exécuté selon la description qui suit pour la détection de la vimentine modifiée. Ainsi, à partir de l'extrait protéique issue de la biopsie, les puits de la plaque de dosage ELISA sont remplies avec des dilutions croissantes de l'échantillon. Les différentes protéines de l'extrait se fixent par adsorption sur le fond des puits. Après lavages des puits, les protéines adhérentes sur les parois des puits sont mises en contact avec les anticorps spécifiques destinés à détecter la présence de la vimentine modifiée. De ce fait, l'anticorps anti-vimentine modifiée s'immobilisera dans le fond du puits, si et seulement si la protéine antigène est adsorbée sur la paroi de la cupule. Une étape de détection (basée par exemple sur un test colorimétrique simple) de la présence d'anticorps au fond des puits renseigne par voie de conséquence sur la présence de vimentine modifiée, adsorbée dans les puits et donc sur sa présence dans l'échantillon de départ. La réalisation de dosages sur les dilutions de l'échantillon permet d'estimer de manière quantitative le taux de vimentine modifiée dans l'échantillon biologique. Les analyses des tests ELISA peuvent être réalisées sur des appareils tel que le système VIDAS distribué par Bio-Mérieux (www.biomerieux.com).
b) méthodes basées sur les « micro-arrays de protéines ». Dans une approche similaire d'immobilisation de complexe-immuns, la technique des micro-arrays repose sur une logique de miniaturisation, automatisation et parallélisation plus ou moins massive du nombre de tests. Pour ces tests, il est nécessaire de créer des « micro-arrays de protéines » constitués de surfaces solides généralement planes (lames de verre, fragments de silicium...) comportant des anticorps fixés par divers procédés chimiques sur le support, chaque anticorps étant déposé sur une petite surface du support représentant quelques micron-carrés de surface. Par exemple, les anticorps sont immobilisés sur le support par dépôt de microgouttes de suspension des anticorps sur ces supports (Peluso et al, 2003, Kusnezow et Hoheisel, 2003). Après incubation avec les échantillons à analyser, les complexe-immuns formés peuvent être détectés par divers moyens techniques, les plus courants reposant sur la détection de signaux de fluorescence ou par une méthode faisant appel à la résonance plasmonique de surface (Vikinge et al, 1998 ; Kusnezow et Hoheisel, 2003).
   La résonance plasmonique de surface repose sur un principe physique expérimental bien connu. La surface plane d'un film d'or réfléchit un rayon lumineux incident dans une direction prédite par les lois de l'optique classique. Néanmoins pour une toute petite partie du faisceau lumineux réfléchi, sous une certaine incidence, on constate une diminution significative du nombre de photons réfléchis. L'angle d'incidence de la zone de réflexion de la zone moins lumineuse dépend de la quantité de matière fixée sur la face du film d'or opposée à la face irradiée par le faisceau lumineux incident. Toute interaction d'anticorps, d'antigènes ou constitution de complexe-immuns sur la face opposée d'un détecteur basée sur la résonance plasmonique et irradiée sous une certaine incidence par un rayon lumineux provoque donc un changement sensible de l'angle de réflexion de la partie moins lumineuse du faisceau de lumière réfléchi. La détection de cette déflexion de l'angle de réflexion permet de mettre en évidence et de quantifier la fixation de composants sur le détecteur.
   Cette dernière méthode de détection est à la base d'une technique d'analyse des interactions entre anticorps et protéines et permet de mesurer les paramètres de cinétique d'interaction entre antigènes et anticorps de même que les quantités d'antigènes présents dans un échantillon (Fagerstam et al, 1990 ; Szabo et al, 1995). Cette technique est développée sous la forme d'automates de mesure dont un exemple est connu sous le nom de BIAcore (BIAcore AB, Uppsala, Suède ; http://www.biacore.com). Il est intéressant de mentionner, à titre d'exemple, que l'utilisation de ce type de détecteur pour la détection de phénomènes d'association de fragments peptidiques de la vimentine entre eux, de même que la mise en évidence de l'impact de la phosphorylation sur ces interactions, a fait l'objet d'une publication scientifique récente (Gohara et al, 2001).
c) méthode basée sur la spectrométrie de masse. Enfin, et tout naturellement, la détection de la vimentine modifiée grâce à la technique SELDI peut être déclinée en faisant appel à des anticorps immobilisés sur les supports d'affinité utilisables sur le spectromètre de masse Ciphergen ou tout autre spectromètre de masse approprié pour ce genre d'analyse. Il est en effet possible d'immobiliser des anticorps par greffage chimique sur les supports de silice ou polymères et d'utiliser ces supports pour piéger la vimentine modifiée qui est présente dans un échantillon à analyser. Les complexe-immuns ainsi immobilisés sur les supports peuvent être ensuite analysés par spectrométrie de masse. Dans ce genre d'analyse, le pic de la protéine vimentine modifiée et/ou non-modifiée (tout dépend des anticorps greffés sur le support) peut être détecté dans le spectre de masse. L'utilisation des anticorps greffés sur le support permet de constituer une méthode de dosage extrêmement sensible et spécifique. Compte tenu de l'affinité des anticorps pour la protéine, des quantités infimes de vimentine présentes dans un échantillon pourront être détectées, de plus, la parfaite sélectivité des anticorps vis-à-vis de la protéine contre laquelle ils sont dirigés est une garantie de la totale spécificité du dosage.
d) méthode basée sur le Western-blot (Towbin et al, 1979 ; Burnette 1981). Dans le cas de cette méthode, l'extrait protéique est analysé par migration électrophorétique en gel de polyacrylamide en milieu dénaturant (technique connue dans une de ces variantes sous l'acronyme de SPAGE : pour SDS polyacrylamide gel electrophoresis ; ou électrophorèse en gel de polyacrylamide en présence de l'agent détergent dénaturant Sodium Dodécyl Sulfate). Cette migration électrophorétique permet de séparer les protéines en fonction de leurs masses moléculaires respectives (Laemmli 1970). A l'issue de cette séparation, des protocoles dits de Western-blot, très classiques, permettent d'immuno-détecter au sein du profil d'électrophorèse la protéine d'intérêt à l'aide d'un anticorps dirigé contre cette protéine. Ces protocoles permettent de révéler par des techniques radioactives, fluorescentes ou luminescentes la présence de la protéine d'intérêt. L'analyse peut être menée de manière à accéder à une estimation quantitative suffisamment précise du taux de protéine dans l'échantillon de départ pour réaliser un diagnostic d'intérêt clinique (Procaccio et al, 1999). La réalisation de ce test à l'aide d'un anticorps anti-vimentine modifiée conduira à la révélation sur le profil de Western-blot et pour les échantillons qui contiennent de la vimentine modifiée d'un signal présent au niveau d'une zone de masse moléculaire correspondant à la vimentine modifiée. Ce genre de tests pourrait être réalisé en utilisant les équipements nécessaires à la réalisation de Western-blots distribués par Immunetics (Boston, MA, USA ; http://www.immunetics.com).

Accessoirement, si les conditions de migrations électrophorétiques peuvent être optimisées de sorte que, compte tenu des caractéristiques physico-chimiques différentes (pI et masses moléculaires différentes) de la vimentine et de la vimentine phosphorylée, ces deux composants peuvent être séparés sur deux zones différentes du gel, alors la détection pourrait ne nécessiter dans ce cas qu'un seul anticorps. En effet, dans ce cas, un anticorps reconnaissant à la fois la forme phosphorylée et la forme non-phosphorylée conduira à la manifestation de un ou deux signaux de détection de vimentine sur le Western-blot ; chacun des signaux étant alors facilement attribuable à la vimentine native ou la vimentine phosphorylée selon les distances de migration de ces deux entités constatées lors de l'électrophorèse dans le gel de polyacrylamide.

Dans toutes les méthodes évoquées ci-dessus, il est fait mention de l'utilisation d'anticorps. Cette description privilégiée de l'utilisation d'anticorps dans les méthodes décrites n'exclut pas que les tests évoqués ici puissent être aussi réalisés avec d'autres réactifs capables d'interaction avec la vimentine, modifiée ou non, tels que, à titre d'exemple les aptamères.

Exemple de test de migration électrophorétique avec mise en évidence des modifications post-traductionnelles de la vimentine n'utilisant pas les anticorps pour la détection : de manière semblable à la méthode dite Western-blot décrite ci-dessus, il est possible de procéder à la séparation des protéines dans un gel de polyacrylamide puis après migration de colorer ce gel ou son empreinte, à l'aide de réactifs chimiques spécifiques. Dans ce cas, l'utilisation d'un réactif qui se fixe sélectivement sur des groupements phosphates présents sur des protéines ou induit des réactions chimiques spécifiques avec ces groupements phosphates permettra de produire selon le cas, un signal radioactif, luminescent, colorimétrique (cas du réactif colorant inclus dans le kit «GelCode® phosphoprotein staining kit » - Pierce, Rockford, IL, USA ; hrip://www.piercenet.com) ou fluorescent (cas du fluorochrome inclus dans le kit « Pro-Q® Diamond phosphoprotein gel stain » - Molecular Probes, Eugene, OR, USA ; http://www.probes.com). La création de ce signal renseignera spécifiquement sur la présence de groupements phosphates dans les protéines séparées dans le gel d'électrophorèse. La détection de ce signal révélant la présence de groupes phosphates au niveau d'une protéine qui présente une distance de migration dans le gel compatible avec la masse moléculaire attendue pour la vimentine sera une information suffisante pour permettre un diagnostic de présence de la vimentine modifiée dans l'échantillon biologique.

Telle que décrite ci-dessus, la détection sélective de la vimentine modifiée peut donc également être obtenue par interaction avec des anticorps ou d'autres réactifs capables d'interagir de manière spécifique avec la vimentine ou par la mise en évidence de caractéristiques physico-chimiques originales et distinguables de la protéine.

Par ailleurs, en complément, et comme c'est déjà le cas en ce qui concerne la méthode Western-blot, il est envisageable de réaliser des tests permettant de révéler ou d'accéder à l'évaluation de deux ou plusieurs paramètres qui combinés permettent de confirmer la présence de la vimentine phosphorylée dans un échantillon. Ces combinaisons de paramètres peuvent être par exemple : la taille et la détection de groupes phosphates ; la taille et la détection à l'aide de composants interagissant spécifiquement avec la vimentine ; le point isoélectrique et la taille de la protéine... Des analyses par électrophorèse bi-dimensionnelles sont potentiellement réalisables en exploitant les instruments et ré actifs de la gamme Multiphor™ par exemple, mise au point par Amersham (http://wwwl.amershambiosciences.com).

En outre, considérant que la vimentine phosphorylée constitue un marqueur très informatif qui permet de pronostiquer l'évolution favorable ou non d'une tumeur, la méthode de diagnostic et de pronostic pathologique peut inclure ou reposer sur la détection voire le dosage des activités enzymatiques de divers catalyseurs du métabolisme cellulaire telles que, notamment, les kinases et/ou phosphatases qui interviennent dans le processus de modification post-traductionnelle par phosphorylation de la vimentine.

Il est en effet tout à fait envisageable de concevoir une méthode d'intérêt diagnostique et pronostique ne reposant pas forcément sur la détection de la seule vimentine phosphorylée mais sur les composants essentiels voire spécifiques qui concourent à la modification et/ou la stabilité de la vimentine sous sa forme phosphorylée. La méthode de diagnostic reposerait dans ce cas sur la détection d'un ou plusieurs des composants cellulaires qui agissent dans ce processus de modification de la vimentine (ces composants sont à rechercher dans une liste non exhaustive des facteurs suivants : kinases ; phosphatases ; protéases ; transporteurs facilitant la présentation de la vimentine aux kinases dans des compartiments cellulaires spécifiques ; transporteurs assurant la séquestration, et par voie de conséquence la stabilité, de la vimentine phosphorylée dans des compartiments sub-cellulaires particuliers voire extra cellulaires).

A titre d'exemple, le dosage dans les échantillons biopsiques ou autres fluides biologiques de l'activité de phosphorylation de la, ou des kinases qui phosphorylent la vimentine pourraient montrer une activité nulle ou normale de ces kinases dans le cas de tumeurs invasives et au contraire, une augmentation de l'activité de phosphorylation détectable, plus ou moins intense, dans le cas de tumeurs non-invasives. Inversement, et selon la même logique que précédemment, il est facilement prédictible que le dosage des phosphatases qui éliminent les groupements phosphates modifiant les acides aminés de la vimentine pourraient montrer des activités normales dans le cas de tumeurs non-invasives et au contraire, une augmentation de l'activité de déphosphorylation détectable dans le cas de tumeurs invasives.

En outre, non seulement, la mesure des activités enzymatiques de ces composés pourrait constituer une méthode diagnostique d'invasivité des tumeurs mais la mise en évidence physique de ces composés couplée à leur estimation quantitative, constituerait tout autant une méthode acceptable d'analyse si tant est que : soit la quantité de ces composants est modifiée dans la cellule ou soit que l'une ou l'autre de leurs caractéristiques physico-chimiques sont altérées dans ces cellules tumorales invasives ou non et que ces altérations puissent être détectables à l'aide d'une technique appropriée.

Ici également, il peut être fait mention d'une méthode de détection basée sur la révélation des capacités de phosphorylation propres à un échantillon tumoral :
ie : sur un support type micro-array, on peut déposer un certain nombre de peptides connus pour être des cibles de kinases. Sur ce micro-array, on peut déposer divers peptides cibles ET les peptides correspondant aux peptides de la vimentine où se produisent les phosphorylations (détectés dans le cadre de la présente invention). En incubant ce micro-array avec un lysat de cellules à analyser, en présence d'ATP radio-marqué, on va révéler la compétence de l'échantillon biologique à phosphoryler tels ou tels peptides. De ce fait, on identifie globalement la composition qualitative (et semi-quantitative) de l'échantillon en ces composants de type kinases actives.

On réalise donc un « phosphorylome » de l'échantillon. L'existence de kinases spécifiques de la vimentine (ou en quantité altérée par rapport à une référence de tissu sain) permettra d'établir un pronostic d'invasivité ou non-invasivité de la tumeur dont un échantillon a été étudié avec ce procédé.

Le procédé selon l'invention peut être employé pour la détermination de l'agressivité et/ou de l'invasivité de tout type de tumeur associée à une surexpression de vimentine, notamment les tumeurs du système nerveux central, les carcinomes (tumeurs épithéliales) mammaires hormone-indépendants, les carcinomes du col de l'utérus, les carcinomes du rein, les cancers de la prostate, les métastases osseuses, les hépatocarcinomes les mélanomes ou de cancers mammaires.

De manière avantageuse, le procédé selon l'invention est employé pour la détermination de l'agressivité et/ou de l'invasivité des tumeurs du système nerveux central, notamment les astrocytomes et les méningiomes.

La présente invention concerne également l'utilisation d'un kit de diagnostic pour la mise en oeuvre d'un procédé selon l'invention, caractérisé en ce qu'il comprend sur un support approprié, un moyen permettant la détection de la vimentine non phosphorylée et/ou un moyen permettant la détection de la vimentine phosphorylée.

Selon un premier mode de réalisation de l'invention, le moyen de détection de la vimentine non phosphorylée est choisi parmi :
Pour les tests basés sur l'utilisation de la spectrométrie de masse, le kit nécessitera des supports d'affinité pour la capture de la protéine (exemple : barettes ProteinChip® de Ciphergen). Ces supports seront adaptés en fonction du mode de capture choisi : supports à réactivité chimique adaptée à la fixation de la vimentine, ou anticorps monoclonaux ou polyclonaux reconnaissant tout ou partie de la protéine ou de ses peptides. Les réactifs de type phosphatase et/ou protéase seront également nécessaires selon le protocole de réalisation choisi.
Pour les autres tests, notamment tests ELISA, les anticorps polyclonaux ou monoclonaux réagissant contre tout ou partie de la vimentine seront indispensables. Des réactifs spécifiques pour la détection des complexe-immuns formés lors du test seront choisis parmi les systèmes de détection classique appropriés à ce genre de tests (par exemple anticorps secondaires couplés à des systèmes enzymatiques autorisant l'exécution de réaction colorimétrique). Les réactifs de type phosphatase et/ou protéase seront également nécessaires selon le protocole de réalisation choisi.
Pour les tests de radio-immunoessais, outre les anticorps (ou autres agents de capture spécifiques de la vimentine et ses dérivés), un traceur identique à la vimentine, ou mimant celle-ci vis-à-vis des agents de capture, sera requis sous forme marquée radioactivement (ou éventuellement selon d'autres procédés, par exemple : marquée de manière fluorescente).

Selon un autre mode de réalisation de l'invention, le moyen de détection de la vimentine phosphorylée est choisi parmi :
Pour les tests basés sur l'utilisation de la spectrométrie de masse, le kit nécessitera des supports d'affinité pour la capture de la protéine (exemple : barettes ProteinChips de Ciphergen). Ces supports seront adaptés en fonction du mode de capture choisi : supports à réactivité chimique adaptée à la fixation de la vimentine, ou anticorps monoclonaux ou polyclonaux reconnaissant tout ou partie de la protéine ou de ses peptides. Les réactifs de type protéase seront également nécessaires selon le protocole de réalisation choisi.
Pour les autres tests, notamment tests ELISA, les anticorps polyclonaux ou monoclonaux réagissant contre tout ou partie de la vimentine seront indispensables. Des réactifs spécifiques pour la détection des complexe-immuns formés lors du test seront choisi parmi les systèmes de détection classique appropriés à ce genre de tests (par exemple anticorps secondaires couplés à des systèmes enzymatiques autorisant l'exécution de réaction colorimétrique). Les réactifs de type protéase seront également nécessaires selon le protocole de réalisation choisi. Pour le Western-blot : anticorps spécifiques de la protéine ou de ses peptides ou réactifs de marquage colorimétriques ou fluorescents spécifiques des groupements phosphoryles.
Pour les tests de radio-immunoessais, outre les anticorps (ou autres agents de capture spécifiques de la vimentine et ses dérivés), un traceur identique à la vimentine, ou mimant celle-ci vis-à-vis des agents de capture, sera requis sous forme marquée radioactivement (ou éventuellement selon d'autres procédés, par exemple : marquée de manière fluorescente).

De manière avantageuse, le support est choisi parmi des supports de composés chimiques capables d'interagir avec la vimentine phosphorylée ou non-phosphorylée (par exemple : supports de silice à capacité d'échanges d'ions), des plaques de dosages multi-puits de matière plastique, des gels de polyacrylamide ou autre matrice pour la séparation des protéines selon leurs caractéristiques physico-chimiques (masse moléculaire, charges ioniques,...), des billes de latex ou autres matériaux activées de manière appropriée par les réactifs autorisant des interactions avec la vimentine ou les complexe-immuns.

De manière évidente pour l'homme de l'art, le kit diagnostic pourra inclure également tout type de réactifs chimiques indispensables à l'extraction, solubilisation, enrichissement, digestion enzymatique de la protéine phosphorylée ou non-phosphorylée ainsi que pour la mise en évidence de la protéine (coloration, modifications chimiques,...). Le kit contiendra aussi pour des besoins de quantification, et de contrôle-positifs, des protéines standard appropriées, apparentées ou non à la vimentine phosphorylée ou non-phosphorylée.

La présente invention concerne aussi un procédé pour identifier un agent anticancéreux permettant de réduire l'agressivité et/ou l'invasivité d'une tumeur en favorisant la phosphorylation de la vimentine, caractérisé en ce qu'il comprend les étapes de :
- mise en culture de cellules produisant de la vimentine non phosphorylée,
- mise en contact desdites cellules avec un agent à tester,
- analyse de la vimentine produite pour déterminer si elle est phosphorylée ou non après culture des cellules en contact avec l'agent à tester,
- sélection du ou des agents capables d'induire la phosphorylation de la vimentine dans les cellules cancéreuses testées.

L'homme du métier saura déterminer les moyens nécessaires à la mise en oeuvre du procédé selon les agents testés susceptibles d'agir sur la phosphorylation de la vimentine : « petites molécules », siRNA, antisens, etc.

De manière avantageuse, les cellules produisant de la vimentine sont des cellules cancéreuses. Il peut également s'agir de cellules non cancéreuses exprimant la vimentine ou encore de cellules recombinantes, plus particulièrement de cellules de mammifères, comprenant un vecteur permettant l'expression de la vimentine ou ses fragments.

De manière également avantageuse, l'analyse de la vimentine produite, permettant de déterminer si elle est phosphorylée ou non, est effectuée par le procédé défini précédemment

Un agent approprié peut être utilisé pour favoriser la phosphorylation de la vimentine dans les cellules cancéreuses, pour la préparation d'un médicament destiné au traitement des tumeurs cancéreuses.

Différents moyens peuvent être employés à cet effet, selon différents axes thérapeutiques :
1. La première stratégie envisageable consiste à intervenir directement sur les concentrations cellulaires des différentes formes de vimentine. Dans cette optique, 3 axes d'intervention sont possibles :
   a. abaisser directement la quantité de vimentine native (non-phosphorylée) synthétisée par les cellules ou en réduire sa bio-disponibilité au sein de la cellule et permettre ainsi d'accroître le rendement global de phosphorylation enzymatique de la vimentine cellulaire. Ceci peut être atteint par la modification du taux d'expression de la vimentine, ou par la séquestration, voire « neutralisation » de la vimentine non-phosphorylée. Cette dernière possibilité reposerait sur l'utilisation de composés qui vont se complexer et « masquer » cette protéine, ou sur le contrôle de l'adressage de la protéine hors du compartiment cytosolique (routage dans des compartiments subcellulaires spécifiques ou éventuellement sécrétion). Ces deux opérations indépendantes ayant pour objectif de soustraire la vimentine native à toute implication dans les phénomènes biologiques de la cellule.
   b. favoriser l'accessibilité de la vimentine aux kinases. En agissant sur la disponibilité de certains composants cellulaires qui interagissent de manière naturelle avec la vimentine, il peut être possible de stimuler la phosphorylation de cette protéine.
   c. augmenter la stabilité et la durée de vie de la vimentine phosphorylée en contrariant les systèmes cellulaires qui participent à la dégradation ou à l'élimination cytoplasmique de celle-ci.
2. Une deuxième possibilité consiste à promouvoir directement l'action des kinases qui phosphorylent la vimentine. Ceci peut être envisagé de deux façons différentes : par l'augmentation de l'activité enzymatique spécifique de ces kinases, ou par leur surproduction,
3. La troisième approche qui est susceptible de favoriser l'accumulation de phospho-vimentine est basée, à l'inverse de la stratégie décrite au point numéro 2 ci-dessus, sur des actions diverses destinées à réduire le taux de phosphatases cellulaires ou leur activité.

Dans un souci d'efficacité thérapeutique optimale, une combinaison de deux ou plusieurs de ces approches n'est bien évidemment pas exclue.

### Stratégie 1 : Modifications de paramètres relatifs à la vimentine (taux d'expression, accessibilité, stabilité).

Dans le cadre de cette stratégie, il est techniquement possible d'envisager de réduire la concentration cellulaire de vimentine par le blocage plus ou moins partiel de l'expression du gène par différents moyens. Une possibilité repose sur la régulation négative de l'expression du gène ; l'autre sur l'activation de la dégradation des ARN messagers (ARNm) ou sur la réduction de l'efficacité de leur traduction.

Divers composés ou molécules pharmacologiques peuvent s'avérer capables de réduire plus ou moins sélectivement l'efficacité de transcription du gène de la vimentine. Ceci peut être obtenu en particulier en modifiant quantitativement ou qualitativement le rôle joué par des régions génétiques essentielles sur le taux de transcription du gène de la vimentine (régions promotrices, enhancers, silencers...). A titre d'exemple, des composés tels que les dérivés de l'acide rétinoïque ont déjà été montrés capables de freiner la transcription du gène de la vimentine et des dérivés de l'acide rétinoïque (N-(4-hydroxyphenyl) retinamide ou 4-HPR) bloqueraient la carcinogenèse prostatique (Webber et al, 1999).

Le contrôle de l'expression du gène peut également se concevoir en altérant la stabilité et/ou les possibilités de traduction de l'ARNm, produit de la transcription normale du gène.

Cette stratégie implique par exemple l'utilisation d'un ou de plusieurs polynucléotides-antisens, capables d'interagir dans la cellule avec l'ARNm et de perturber sa stabilité ou sa capacité à être traduit en vimentine par les ribosomes. L'utilisation de polynucléotides antisens a déjà été expérimentée *in vitro* pour abaisser l'expression de la vimentine. Ainsi dans une lignée de cellules tumorales de prostate, hormone-insensible et invasive, il a été observé une surexpression du gène de la vimentine. La transfection avec une construction antisens-vimentine réduit effectivement le caractère invasif de ces tumeurs mesuré *in vitro* (Singh et al, 2003). Ce genre de construction oligonucléotides antisens-vimentine réduit aussi la vitesse de migration de cellules épithéliales au cours de la cicatrisation de lésions (Gilles et al, 1999). De même, la transfection *in vitro* de cellules malignes de carcinome mammaire avec des oligonucléotides anti-sens destinés à bloquer l'expression de la vimentine concourt à réduire l'activité invasive des cellules ainsi traitées (Hendrix et al, 1997). Dans les travaux cités ci-dessus, l'impact des composés polynucléotidiques ou constructions antisens sur le taux de phosphorylation de la vimentine n'a pas été déterminé.

L'utilisation appropriée d'ARNi (ARN d'interférence, siRNA) (Fire et al, 1998 ; Bernstein et al, 2001 ; Elbashir et al, 2001 a et b), judicieusement sélectionnés en fonction de la séquence primaire du transcrit codant la vimentine, est bien évidemment une solution à l'efficacité attendue pour abaisser le taux de transcrit disponible dans la cellule pour la synthèse de vimentine.

Enfin, selon une troisième option, la stabilité de l'ARNm peut également être réduite en intervenant sur la concentration et/ou l'affinité des composés cellulaires qui interagissent avec l'ARNm de la vimentine. En effet, la région 3' non-codante du transcrit de la vimentine interagit avec des protéines telles que HAX-1 et eEF1-gamma (Al-Maghrebi et al, 2002). Il est reconnu que la fixation de protéines sur les régions non-codantes des ARNm est de nature à modifier sensiblement la stabilité des ARNm (Saini et al, 1990 ; Cuadrado et al, 2002). Il est donc tout à fait plausible d'envisager que les modifications de concentration de ces protéines ou l'utilisation de molécules chimiques ayant la vertu de modifier la force d'interaction de ces protéines avec l'ARNm de la vimentine pourront de manière indirecte, affecter la stabilité du transcrit et par voie de conséquence aboutir à la baisse de synthèse de la vimentine.

Une autre possibilité consiste à créer, dans les cellules, des complexes entre la vimentine et des composés moléculaires variés pour limiter la quantité de vimentine libre non-modifiée post-traductionnellement, et faciliter de ce fait un taux de phosphorylation important de cette protéine par les kinases. Evénement final dont il est attendu, au niveau cellulaire, une limitation de l'invasivité des tumeurs. Les molécules qui pourraient interagir avec la vimentine non-modifiée sont des molécules chimiques, des anticorps ou des aptamères (molécules polypeptidiques ou polynucléotidiques capables de se fixer avec une forte affinité sur une protéine, en l'occurrence la vimentine ici). La vimentine est une molécule antigénique et la production d'anticorps dirigés contre cette protéine est tout à fait réalisable. Des travaux tels que ceux publiés par Hartmann et al, (2004) montrent d'ailleurs que la vimentine peut même susciter des réactions auto-immunes puisque des auto-anticorps dirigés contre la vimentine peuvent être détectés dans le sérum de nombreux patients atteints de lymphomes cutanés.

L'intervention sur la séquestration de la vimentine dans des compartiments subcellulaires ou la modification de son excrétion par des systèmes de pompage transmembranaires reste encore une hypothèse de travail. Néanmoins, cette approche constitue une piste intéressante puisqu'il a été rapporté que les macrophages se montraient capables de sécréter la vimentine phosphorylée (Mor-Vaknin et al, 2003). Il existe donc bien des systèmes transporteurs capables de véhiculer la vimentine du cytosol vers l'extérieur des cellules.

Il est également envisageable de modifier les modalités d'interaction de composés cellulaires normaux avec la vimentine. Il s'agit dans ce cas de modifier les interactions entre la vimentine et les protéines ou composés cellulaires normaux avec lesquels elle forme naturellement des complexes.

A titre d'exemple, on peut mentionner que la vimentine peut interagir avec la protéine dite 14-3-3 des cellules tumorales. L'association de 14-3-3 avec la phosphovimentine altérerait la déphosphorylation de cette dernière (Tzivion et al, 2000). De ce fait, toute action susceptible de perturber l'interaction de la vimentine avec cette protéine 14-3-3 (ou d'autres composés qui se fixent sur la vimentine) aura la potentialité de faciliter l'accumulation de la phosphovimentine et donc par voie de conséquence de réduire de manière contrôlable l'invasivité des tumeurs.

Enfin, tous éléments cellulaires tels que des protéases, qui dégraderaient spécifiquement la vimentine phosphorylée, ou des transporteurs membranaires qui excréteraient spécifiquement la vimentine, représenteraient des cibles contre lesquelles dédier des molécules ou approches destinées à bloquer de telles activités. L'inhibition de ces activités pourrait globalement tendre vers la diminution de la concentration cellulaire de vimentine phosphorylée.

La deuxième stratégie impliquant une activation de l'activité de phosphorylation de la vimentine repose sur le développement de molécules susceptibles d'interagir directement ou non avec les kinases et à stimuler leur activité spécifique. Il est intéressant de constater dans cette logique que le traitement des cellules CHO-K1 (cellules d'origine fibroblastique pérennisées à partir de biopsie ovarienne d'hamster chinois) avec des dérivés de l'AMPc (adénosine 3',5'-monophosphate cyclique) induit la perte du phénotype malin. Cet événement va de pair avec la phosphorylation de la vimentine (Chan et al, 1989). Il est possible de supposer que dans le cas de cet exemple, l'AMPc pourrait être tenu pour responsable, sans que cela ait été démontré formellement, de l'activation de kinases dépendantes de l'AMPc. Par ailleurs, l'augmentation du taux de kinases cellulaires peut être augmenté par diverses approches. Par exemple, les technologies de biologie moléculaire offrent la possibilité de créer des constructions géniques pour l'expression de kinases dans les cellules tumorales après import de ces constructions dans les dites cellules.

Enfin, en ce qui concerne la troisième stratégie, les phosphatases peuvent constituer une cible moléculaire de choix. En effet, tout ralentissement de l'activité de ces enzymes devrait conduire à l'accumulation de phosphovimentine. Des molécules spécifiques, dédiées à l'inhibition des phosphatases, seront d'autant plus efficaces que les phophatases spécifiquement impliquées dans la déphosphorylation de la vimentine auront été identifiées. L'inhibition des phosphatases pourra reposer sur l'utilisation de molécules chimiques capables d'interagir avec ces enzymes ou le recours à des ARNi ou constructions antisens capables de perturber l'expression de ces enzymes. A titre d'exemple, on peut mentionner qu'il a été montré que la calyculin A qui bloque la phosphatase de type 1 favorise la détection d'une forme phosphorylée de la vimentine par la Rho kinase au niveau de la sérine 71 de la vimentine (Inada et al, 1999). Les possibilités de contrarier l'activité des phosphatases peuvent aussi impliquer des interventions sur la formation des complexes actifs multimoléculaires de phosphatases. Toute action sur les composés qui activent les phosphatases est susceptible de prévenir l'activation de ces enzymes. Il a été démontré par exemple que la protéine B55 qui active la phosphatase PP2A est requise pour la déphosphorylation de la vimentine (Turowski et al, 1999).

Les exemples de réalisation ci-dessous permettent de mieux illustrer l'invention, sans toutefois chercher à en limiter la portée.

### Exemple 1 : Détection de la vimentine phosphorylée pour le diagnostic de diverses tumeurs solides invasives ou non-invasives selon le mode de réalisation reposant sur la spectrométrie de masse de type SELDI.

Les échantillons de tissus, sous formes de coupes de biopsies congelées sont utilisés pour la préparation des extraits protéiques et analysés selon les protocoles décrits ci-après : Des coupes de tissus de 20 µm d'épaisseur sont réalisées au microtome et 20 coupes sont mises en incubation pendant 30 minutes à 4°C dans 300 microlitres d'un tampon de lyse (Reporter Lysis Buffer, Promega, Madison, WI, USA) contenant un mélange classique d'antiprotéases. L'échantillon est vortexé à plusieurs reprises pendant cette période d'incubation. Après centrifugation (10000g pendant 10 min à 4°C), le surnageant (dit extrait final) est obtenu et un dosage protéique par une méthode classique permet de contrôler que la concentration en protéine dans l'extrait final atteint un titre de 2 à 3 microgrammes de protéines totales par microlitre.
Les extraits finaux protéiques sont analysés grâce aux barrettes d'analyse ProteinChip® de type SAX2 de la société Ciphergen composées de surfaces actives composées d'une matrice dite échangeuse d'anions. Pour assurer l'équilibration des charges ioniques des surfaces actives, les ProteinChip® SAX2 sont imprégnées d'un tampon de fixation (Tris 100mM pH 8,0 et 0, 1 % Triton X100) pendant 5 minutes. Après élimination de ce tampon les extraits finaux protéiques sont déposés sur les surfaces actives de la manière suivante : les extraits sont dilués à un titre final de 0,1 microgramme par microlitre et 100 microlitres de cette dilution sont déposés au contact des surfaces actives. Les ProteinChip® sont ensuite mises en incubation à température ambiante pendant 45 minutes et soumises à une agitation vigoureuse permanente. Après incubation, les ProteinChip® sont lavées deux fois à l'aide d'un tampon constitué de Tris 100mM pH 8,0 et 0,1% Triton X100, puis une fois avec un deuxième tampon de lavage composé de Tris 100mM pH 8,0. Un dernier lavage est réalisé en tampon HEPES 2mM, pH 7,5. Enfin, les surfaces actives sont séchées sous courant d'air et 1,6 microlitre d'une solution SPA (Sinapinic acid, Ciphergen) est déposé sur ces surfaces.

L'analyse des ProteinChip® est réalisée à l'aide du spectromètre de masse Ciphergen. L'appareil est soumis à une étape de calibration utilisant des standards protéiques purifiés connus et déposés sur des ProteinChip®. Les standards habituellement utilisés sont l'insuline bovine (masse moléculaire 5733,6 Da) le cytochrome c bovin (12230,9 Da) et l'albumine sérique bovine (66410,0 Da).

L'analyse d'échantillons biopsiques selon la méthode décrite ci-dessus a été effectuée sur des tumeurs invasives ou non-invasives de type méningiomes (35 tumeurs), sur des tumeurs du poumon (4 échantillons) et des tumeurs des cellules gliales du système nerveux central (type glioblastomes et oligodendrogliomes, 4 échantillons). La vimentine phosphorylée est détectée par la présence d'un pic à 53,5 kDa.

Les résultats d'analyse sont rassemblés dans le tableau 1 (valeurs calculées sur un ensemble de 35 tumeurs différentes).

| Type d'échantillon (nombre) | Intensité du pic de masse détecté à 53,5 kDa (moyenne des intensités en unités arbitraires) | P -value |
|---|---|---|
| Extrait de tumeur non invasive (17) | 6,5 | 0,00024 |
| Extrait de tumeur invasive (18) | 1 | |

### Exemple 2 : Détection de la vimentine non-phosphorylée

La vimentine phosphorylée peut être purifiée des extraits protéiques par un protocole qui consiste à soumettre l'extrait à une chromatographie sur colonne Q hyperD® dans des conditions opératoires facilitant la fixation de la vimentine sous sa forme phosphorylée sur le support chromatographique puis à éluer spécifiquement cette protéine par des solutions de composition bien définie. L'extrait est mis en incubation avec la résine de la colonne en présence de Tris 100 mM pH 8,5 pendant 30 min avec agitation. On réalise 3 lavages de 5 min en tampon Na-citrate 100mM, pH 3,5 avec agitation puis 3 lavages dans une solution composé de 5% Acétonitrile + 0,5% acide trifluoroacétique + 50% Isopropanol. L'élution de la protéine d'intérêt est réalisée en 16% Acétonitrile + 0,1% acide trifluoroacétique + 33% Isopropanol.

Après purification de la protéine phosphorylée, une incubation en présence d'une phosphatase selon des méthodes conventionnelles (pendant 2 heures à température ambiante en présence de phosphatase alcaline extraite d'intestin de veau) permet d'éliminer les groupements phosphates. La détection de la vimentine non-phosphorylée peut être réalisée par spectrométrie de masse SELDI.

La vimentine phosphorylée est capable d'interagir de manière extrêmement forte avec une matrice d'échange d'anions type ProteinChip® SAX2. L'adsorption de la vimentine sur la matrice résiste à des traitements par des tampons de pH acide et la protéine fixée n'est pas éluée, en particulier par lavage à pH 3,5. Après traitement par une phosphatase de la vimentine phosphorylée purifiée à partir des tumeurs non-invasives, la vimentine dé-phosphorylée perd son caractère anionique et n'est plus capable d'adsorption sur la matrice échangeuse d'anions SAX2. Elle reste toutefois capable de se fixer sur une matrice hydrophile (Ciphergen ProteinChip® NP20).

Les résultats d'analyse par spectrométrie de masse après fixation sur support ProteinChip® Ciphergen de type SAX2 et NP20 sont indiqués dans le tableau 2.

| Support ProteinChip® sélectionné pour l'analyse SELDI | ProteinChip® SAX2 (surface échangeuse d'anions) | ProteinChip® NP20 (surface hydrophile) |
|---|---|---|
| | Détection du pic de masse à 53,5 kDa de la vimentine phosphorylée | Masse du pic détecté (en kDa) |
| Type d'échantillon | | |
| Vimentine phosphorylée purifiée | présent | 53,5 |
| Vimentine phosphorylée purifiée ayant subi un traitement par la phosphatase | absent | 53,2 |

### Exemple 3 : Détection de fragments de digestion enzymatique de la vimentine

La détection de la vimentine peut être effectuée en exploitant la fragmentation protéolytique de l'échantillon ou de la vimentine phosphorylée purifiée (voir exemple 2). Dans ce cadre, l'identification de la protéine repose sur une stratégie consistant à analyser par spectrométrie de masse le résultat de la digestion de la vimentine phosphorylée (voire déphosphorylée par traitement préliminaire aux phosphatases) par une protéase (trypsine, ou endoprotéinase-Lys, ou endoprotéinase Glu-C,...) et à repérer les pics peptidiques détectés comportant des masses identiques à celles des peptides prédits de la structure de la vimentine.

L'analyse du résidu de digestion par l'endoproteinase Glu-C de la vimentine phosphorylée montre par exemple 25 peptides de masses moléculaires différentes dans une fenêtre de détection de masses moléculaires comprises entre 1000 et 3000 Da. L'analyse de correspondance entre la masse de ces 25 peptides détectés avec celles des peptides pouvant être générés à partir de la vimentine a montré que plus d'une douzaine de ces peptides correspondent strictement à des peptides issus de la digestion de la vimentine par l'endoproteinase Glu-C.

Les résultats d'analyse sont indiqués dans le tableau 3

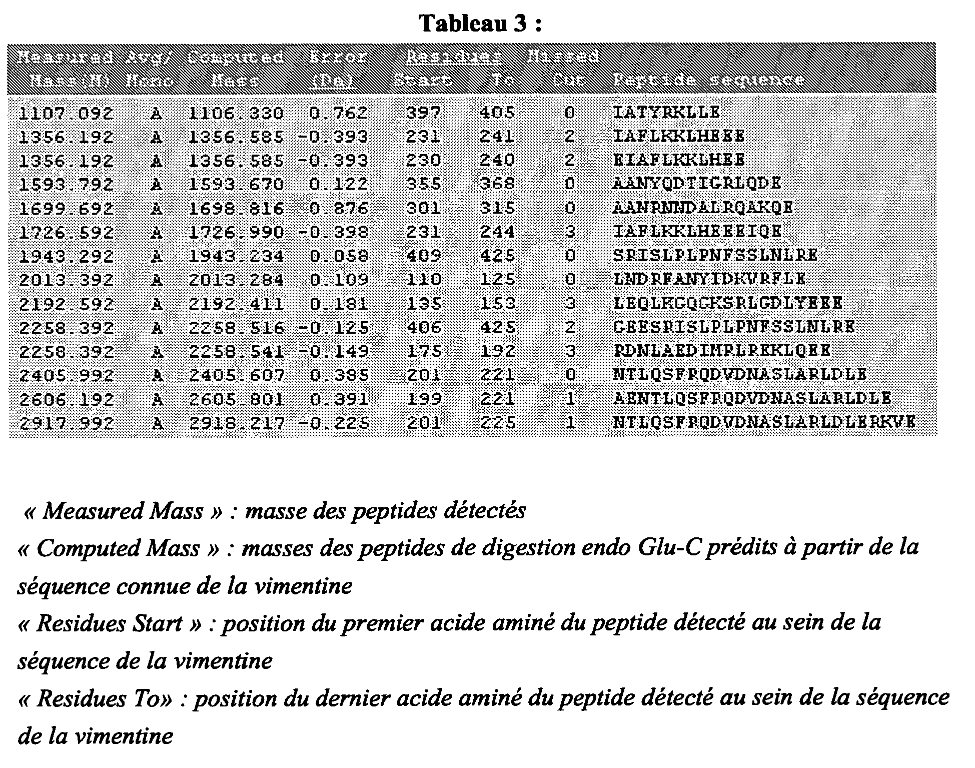

### Références bibliographiques

- Al-Maghrebi M, Brule H, Padkina M, Allen C, Holmes WM, Zehner ZE., Nucleic Acids Res. 2002, 30, 5017-28.
- Ando S, Tanabe K, Gonda Y, Sato C, Inagaki M., Biochemistry. 1989, 28, 2974-9.
- Ando S, Tokui T, Yamauchi T, Sugiura H, Tanabe K, Inagaki M., Biochem Biophys Res Commun. 1991, 175, 955-62.
- Bernstein E, Denli AM, Hannon GJ., RNA. 2001, 7, 1509-21.
- Blomberg K, Hurskainen P, Hemmila I., Clin Chem. 1999, 45, 855-61.
- Booth JC, Hannington G, Bakir TM, Stern H, Kangro H, Griffiths PD, Heath RB., J Clin Pathol. 1982, 35, 1345-8.
- Burnette WN., Anal Biochem. 1981, 112, 195-203.
- Cairncross JG, Ueki K, Zlatescu MC, Lisle DK, Finkelstein DM, Hammond RR, Silver JS, Stark PC, Macdonald DR, Ino Y, Ramsay DA, Louis DN., J Natl Cancer Inst. 1998, 90, 1473-9.
- Cattoretti G, Andreola S, Clemente C, D'Amato L, Rilke F., Br J Cancer. 1988, 57, 353-7.
- Chan D, Goate A, Puck TT., Proc Natl Acad Sci U S A. 1989, 86, 2747-51.
- Chatel M. et Brucher J.-M., dans « Neuro-oncologie », Editeur J. Hildebrand, Doin 2001 ; p 25-34
- Chou YH, Ngai KL, Goldman R., J Biol Chem. 1991, 266, 7325-8.
- Chu YW, Seftor EA, Romer LH, Hendrix MJ., Am J Pathol. 1996, 148, 63-9.
- Cuadrado A, Navarro-Yubero C, Fumeaux H, Kinter J, Sonderegger P, Munoz A., Nucleic Acids Res. 2002, 30, 2202-11.
- Daumas-Duport C. et Figarella-Branger D., dans « Classification histopronostiques des tumeurs cérébrales gliales et leurs impacts en thérapeutique » Editions espaces 34, 2002 ; p 11-48)
- Dierick AM, Praet M, Roels H, Verbeeck P, Robyns C, Oosterlinck W., Histopathology. 1991, 18,315-22.
- Donhuij sen K, Schulz S., Pathol Res Pract. 1989,184,287-91.
- Elbashir SM, Lendeckel W, Tuschl T., Genes Dev. 2001a, 15, 188-200.
- Elbashir SM, Harborth J, Lendeckel W, Yalcin A, Weber K, Tuschl T., Nature. 2001b, 411, 494-8.
- Ellington AD, Szostak JW, Nature 1990, 346, 818-22
- Engvall, P. Perlmann, Immunochemistry 1971, 8, 871-874
- Engvall E, Jonsson K, Perlmann P., Biochim Biophys Acta. 1971, 251, 427-34.
- Engvall E, Perlmann P., J Immunol. 1972, 109, 129-35.
- Enomoto K, Aono Y, Mitsugi T, Takahashi K, Suzuki R, Preaudat M, Mathis G, Kominami G, Takemoto H., J Biomol Screen. 2000, 5, 263-8.
- Fagerstam LG, Frostell A, Karlsson R, Kullman M, Larsson A, Malmqvist M, Butt H., J Mol Recognit. 1990,3,208-14.
- Ferrari S, Battini R, Kaczmarek L, Rittling S, Calabretta B, de Riel JK, Philiponis V, Wei JF, Baserga R., Mol Cell Biol. 1986, 6, 3614-20.
- Ferrari S, Cannizzaro LA, Battini R, Huebner K, Baserga R., Am J Hum Genet. 1987, 41, 616-26.
- Fire A, Xu S, Montgomery MK, Kostas SA, Driver SE, Mello CC., Nature. 1998, 391, 806-11.
- Gao C, Mao S, Lo CH, Wirsching P, Lerner RA, Janda KD., Proc Natl Acad Sci U S A. 1999, 96, 6025-30.
- Gilles C, Polette M, Piette J, Delvigne AC, Thompson EW, Foidart JM, Birembaut P., J Pathol. 1996, 180, 175-80.
- Gilles C, Polette M, Zahm JM, Tournier JM, Volders L, Foidart JM, Birembaut P., J Cell Sci. 1999,112,4615-25.
- Gohara R, Tang D, Inada H, Inagaki M, Takasaki Y, Ando S., FEBS Lett. 2001, 489, 182-6.
- Hartmann TB, Thiel D, Dummer R, Schadendorf D, Eichmuller S., Br J Dermatol. 2004, 150, 252-8.
- Heatley M, Maxwell P, Whiteside C, Toner P., J Clin Pathol. 1995, 48, 1031-4.
- Heatley MK, Ewings P, Odling Smee W, Maxwell P, Toner PG., Pathology. 2002, 34, 230-2.
- Hechemy K, Stevens RW, Gaafar HA., Appl Microbiol. 1974, 28, 306-11.
- Hendrix MJ, Seftor EA, Seftor RE, Trevor KT., Am J Pathol. 1997, 150, 483-95.
- Holck S, Pedersen L, Schiodt T, Zedeler K, Mouridsen H., Virchows Arch A Pathol Anat Histopathol. 1993,422,475-9.
- Honore B, Madsen P, Basse B, Andersen A, Walbum E, Celis JE, Leffers H., Nucleic Acids Res. 1990, 18, 6692.
- Huang TJ, Lee TT, Lee WC, Lai YK, Yu JS, Yang SD., J Protein Chem. 1994,13, 517-25.
- Hulett HR, Bonner WA, Barrett J, Herzenberg LA. , Science. 1969, 166, 747-9.
- Inada H, Togashi H, Nakamura Y, Kaibuchi K, Nagata K, Inagaki M., J Biol Chem. 1999, 274, 34932-9.
- Kiernan JA, Histological and histochemical methods. Theory and practise. Third edition. Oxford : Butterworth-Heinemann 1999.
- Knappik A, Ge L, Honegger A, Pack P, Fischer M, Wellnhofer G, Hoess A, Wolle J, Pluckthun A, Virnekas B., J Mol Biol 2000, 296, 57-86
- Knight ZA, Schilling B, Row RH, Kenski DM, Gibson BW, Shokat KM., Nat Biotechnol. 2003,21, 1047-54.
- Kusnezow W, Hoheisel JD., J Mol Recognit. 2003, 16, 165-76.
- Laemmli UK., Nature. 1970, 227, 680-5.
- Lang SH, Hyde C, Reid IN, Hitchcock IS, Hart CA, Bryden AA, Villette JM, Stower MJ, Maitland NJ., Prostate. 2002, 52, 253-63.
- Lilienbaum A, Legagneux V, Portier MM, Dellagi K, Paulin D., EMBO J. 1986, 5, 2809-14.
- Mathis G., Clin Chem. 1995, 41, 1391-7.
- Merchant M, Weinberger SR., Electrophoresis. 2000, 21, 1164-77.
- Mor-Vaknin N, Punturieri A, Sitwala K, Markovitz DM., Nat Cell Biol. 2003, 5, 59-63.
- Parks DR, Herzenberg LA. , Methods Enzymol. 1984, 108, 197-241.
- Peluso P, Wilson DS, Do D, Tran H, Venkatasubbaiah M, Quincy D, Heidecker B, Poindexter K, Tolani N, Phelan M, Witte K, Jung LS, Wagner P, Nock S., Anal Biochem. 2003, 312, 113-24.
- Perreau J, Lilienbaum A, Vasseur M, Paulin D., Gene. 1988, 62, 7-16.
- Procaccio V, Mousson B, Beugnot R, Duborjal H, Feillet F, Putet G, Pignot-Paintrand I, Lombes A, De Coo R, Smeets H, Lunardi J, Issartel JP., J Clin Invest. 1999, 104, 83-92.
- Raymond WA, Leong AS., J Pathol. 1989, 158, 107-14.
- Rusnak F, Zhou J, Hathaway GM., J. Biomol. Techniques 2002, 13, 228-37
- Saini KS, Summerhayes IC, Thomas P., Mol Cell Biochem. 1990, 96, 15-23.
- Seshadri R, Raymond WA, Leong AS, Horsfall DJ, McCaul K., Int J Cancer. 1996, 67, 353-6.
- Singer JM, Plotz CM, Pader E, Elster SK., Am J Clin Pathol. 1957, 28, 611-7.
- Singh S, Sadacharan S, Su S, Belldegrun A, Persad S, Singh G., Cancer Res. 2003, 63, 2306-11.
- Sommers CL, Walker-Jones D, Heckford SE, Worland P, Valverius E, Clark R, McCormick F, Stampfer M, Abularach S, Gelmann EP., Cancer Res. 1989, 49, 4258-63.
- Szabo A, Stolz L, Granzow R., Curr Opin Struct Biol. 1995, 5, 699-705.
- Szollosi J, Damjanovich S, Matyus L., Cytometry. 1998, 34, 159-79.
- Towbin H, Staehelin T, Gordon J., Proc Natl Acad Sci U S A. 1979, 76,4350-4.
- Tsujimura K, Ogawara M, Takeuchi Y, Imajoh-Ohmi S, Ha MH, Inagaki M., J Biol Chem. 1994,269,31097-106.
- Tuerk C, Gold L, Science 1990, 249, 505-10
- Turowski P, Myles T, Hemmings BA, Fernandez A, Lamb NJ., Mol Biol Cell. 1999, 10, 1997-2015.
- Tzivion G, Luo ZJ, Avruch J., J Biol Chem. 2000, 275, 29772-8.
- Ueda H, Kubota K, Wang Y, Tsumoto K, Mahoney W, Kumagai I, Nagamune T., Biotechniques. 1999,27, 738-42.
- Vikinge TP, Askendal A, Liedberg B, Lindahl T, Tengvall P., Biosens Bioelectron. 1998, 13, 1257-62.
- Webber MM, Bello-DeOcampo D, Quader S, Deocampo ND, Metcalfe WS, Sharp RM., Clin Exp Metastasis. 1999, 17, 255-63.
- Weinberger SR, Morris TS, Pawlak M., Pharmacogenomics 2000, 1, 395-416
- Yalow RS, Berson SA., J Clin Invest. 1960,39,1157-75.
- Yang HY, Lieska N, Shao D, Kriho V, Pappas GD., Mol Chem Neuropathol. 1994, 21, 155-76.
- Yoon WH, Song IS, Lee BH, Jung YJ, Kim TD, Li G, Lee TG, Park HD, Lim K, Hwang BD., Cancer Lett. 2004, 203, 99-105.
- Yung WK, Luna M, Borit A., J Neurooncol, 1985, 3, 35-8.

- NAKAMURA YU ET AL: "Localized phosphorylation of vimentin by Rho-kinase in neuroblastoma N2a cells" GENES TO CELLS, vol. 5, no. 10, octobre 2000, p.823-837.
- KOPEREK OSKAR ET AL: "Value and limits of immunohistochemistry in differential diagnosis of der cell primary brain tumors" ACTA NEUROPATHOLOGICA, vol. 108, no. 1, juillet 2004, p.24-30.
- WO 03/076651

## Revendications

1. Procédé pour déterminer l'agressivité et/ou l'invasivité d'une tumeur, ledit procédé comprenant, sur un échantillon de cellules tumorales préalablement prélevé, les étapes de :
- préparer un extrait biologique comprenant de la vimentine, et
- déterminer si la vimentine ainsi extraite est phosphorylée ou non phosphorylée, dans lequel la présence significative de vimentine phosphorylée est un indice de non-agressivité et/ou non-invasivité de la tumeur.

2. Procédé selon la revendication 1, **caractérisé en ce que** l'on détermine si la vimentine est phosphorylée ou non phosphorylée au moyen d'une méthode sélectionnée parmi le groupe comprenant :
- l'analyse SELDI-TOF
- la méthode FACS ou ses équivalents
- les marquages isotopiques RIA
- le transfert de fluorescence FRET
- la mise en évidence de complexe-immuns
- les méthodes permettant de détecter des réactions antigènes-anticorps comme les méthodes ELISA, les micro-arrays de protéines ou Western-blot
- les tests de migration électrophorétique avec mise en évidence de modifications post-traductionnelles de la vimentine.

3. Procédé selon l'une des revendications 1 ou 2, **caractérisé en ce que** la tumeur est une tumeur du système nerveux central.

4. Utilisation in vitro d'un kit de diagnostic comprenant sur un support approprié, un moyen permettant la détection de la vimentine non phosphorylée et/ou un moyen permettant la détection de la vimentine phosphorylée, pour déterminer l'agressivité et/ou l'invasivité d'une tumeur, dans laquelle la présence significative de vimentine phosphorylée est un indice de non-agressivité et/ou non-invasivité de la tumeur.

5. Utilisation selon la revendication 4, **caractérisée en ce que** le moyen de détection de la vimentine non phosphorylée est choisi parmi :
les anticorps réagissant avec tout ou partie de la protéine, les réactifs autres que les anticorps capables d'interagir de manière spécifique avec la vimentine, les réactifs de modification et/ou clivage de la protéine au niveau des sites de phosphorylation, les traceurs radioactifs.

6. Utilisation selon la revendication 4, **caractérisée en ce que** le moyen de détection de la vimentine phosphorylée est choisi parmi :
les anticorps réagissant avec tout ou partie de la protéine, les réactifs autres que les anticorps capables d'interagir dé manière spécifique avec la vimentine, les réactifs de modification (marquage colorimétrique ou fluorescent) au niveau des sites de phosphorylation, les réactifs et/ou protéases pour le clivage spécifique de la protéine au niveau des sites de phosphorylation, les traceurs radioactifs.

7. Utilisation selon l'une des revendications 4 à 6, **caractérisée en ce que** le support est choisi parmi : les supports de composés chimiques capables d'interagir avec la vimentine phosphorylée ou non-phosphorylée, les plaques de dosages multi-puits de matière plastique, les gels de polyacrylamide ou autre matrice pour la séparation des protéines selon leurs caractéristiques physico-chimiques, des billes de latex ou autres matériaux activés pour interagir spécifiquement avec la vimentine.

8. Procédé pour identifier un agent anticancéreux permettant de réduire l'agressivité et/ou l'invasivité d'une tumeur en favorisant la phosphorylation de la vimentine, **caractérisé en ce qu'**il comprend les étapes de:
- mise en culture de cellules produisant de la vimentine non phosphorylée,
- mise en contact desdites cellules avec un agent à tester,
- analyse de la vimentine produite pour déterminer si elle est phosphorylée ou non après culture des cellules en contact avec l'agent à tester,
- sélection du ou des agents capables d'induire la phosphorylation de la vimentine dans les cellules cancéreuses testées.

9. Procédé selon la revendication 8, **caractérisé en ce que** les cellules produisant de la vimentine sont des cellules cancéreuses.

10. Procédé selon l'une des revendications 8 ou 9, **caractérisé en ce que** l'analyse de la vimentine produite est effectuée par le procédé selon l'une des revendications 1 ou 2.

## Claims

1. Method, for determining the aggressiveness and/or invasiveness of a tumour, said method comprising, on a sample of tumour cells taken beforehand, the steps consisting in:
- preparing a biological extract comprising vimentin, and
- determining whether the vimentin thus extracted is phosphorylated or nonphosphorylated, in which the significant presence of phosphorylated vimentin is an indication of nonaggressiveness and/or noninvasiveness of the tumour.

2. Method according to Claim 1, **characterized in that** it is determined whether the vimentin is phosphorylated or nonphosphorylated, by means of a method selected from the group comprising:
- SELDI-TOF analysis
- the FACS method or equivalents thereof
- RIA isotopic labelling
- FRET fluorescence transfer
- the detection of immunocomplexes
- methods for detecting antigen-antibody reactions, such as ELISA methods, protein microarrays or Western blotting
- electrophoretic migration tests with demonstration of posttranslational modifications of vimentin.

3. Method according to either of Claims 1 and 2, **characterized in that** the tumour is a central nervous system tumour.

4. Use, in vitro, of a diagnostic kit comprising, on an appropriate support, a means for detecting nonphosphorylated vimentin and/or a means for detecting phosphorylated vimentin, for determining the aggressiveness and/or invasiveness of a tumour, in which the significant presence of phosphorylated vimentin is an indication of nonaggressiveness and/or noninvasiveness of the tumour.

5. Use according to Claim 4, **characterized in that** the means for detecting nonphosphorylated vimentin is chosen from:
antibodies that react with all or part of the protein, reagents, other than antibodies, capable of interacting specifically with vimentin, reagents for modification and/or cleavage of the protein at the phosphorylation sites, and radioactive tracers.

6. Use according to Claim 4, **characterized in that** the means for detecting phosphorylated vimentin is chosen from:
antibodies that react with all or part of the protein, reagents, other than antibodies, capable of interacting specifically with vimentin, reagents for modification (colorimetric or fluorescent labelling) at the phosphorylation sites, reagents and/or proteases for specific cleavage of the protein at the phosphorylation sites, and radioactive tracers.

7. Use according to one of Claims 4 to 6, **characterized in that** the support is chosen from:
support of chemical compounds capable of interacting with phosphorylated or nonphosphorylated vimentin, plastic multiwell assay plates, polyacrylamide gels or other matrix for separating proteins according to their physicochemical characteristics, latex beads or other activated materials for specifically interacting with vimentin.

8. Method for identifying an anticancer agent that makes it possible to reduce the aggressiveness and/or invasiveness of a tumour by promoting vimentin phosphorylation, **characterized in that** it comprises the steps consisting in:
- culturing cells that produce nonphosphorylated vimentin,
- bringing said cells into contact with an agent to be tested,
- analyzing the vimentin produced in order to determine whether it is phosphorylated or nonphosphorylated after culturing of the cells in contact with the agent to be tested,
- selecting the agent(s) capable of inducing vimentin phosphorylation in the cancer cells tested.

9. Method according to Claim 8, **characterized in that** the cells that produce vimentin are cancer cells.

10. Method according to either of Claims 8 and 9, **characterized in that** the analysis of the vimentin produced is carried out by means of the method according to either of Claims 1 and 2.

## Patentansprüche

1. Verfahren zur Bestimmung der Aggressivität und/oder Invasivität eines Tumors, wobei das Verfahren auf einer zuvor entnommenen Tumorzellenprobe die Schritte umfasst:
- Vorbereitung eines biologischen Extrakts, das Vimentin umfasst, und
- Bestimmung, ob das derart extrahierte Vimentin phosphoryliert oder nicht phosphoryliert ist, wobei die signifikante Präsenz phosphorylierten Vimentins ein Indiz für die Nicht-Aggressivität und/oder Nicht-Invasivität des Tumors ist.

2. Verfahren nach Anspruch 1, **dadurch gekennzeichnet, dass** bestimmt wird, ob das Vimentin phosphoryliert oder nicht phosphoryliert ist mit einer Methode, die ausgewählt ist aus der Gruppe, die umfasst:
- die SELDI-TOF-Analyse,
- die FACS-Methode oder ihre Äquivalente,
- die isotopischen RIA-Markierungen,
- den FRET-Fluoreszenztransfer,
- den Nachweis von Immunkomplexen,
- die Methoden, die es erlauben, Antigen-Antikörper-Reaktionen nachzuweisen, wie die ELISA-Methoden, die Protein-Mikroarrays oder Western-Blot,
- die elektrophoretischen Migrationstests mit Nachweis posttraduktioneller Modifizierungen des Vimentins.

3. Verfahren nach einem der Ansprüche 1 oder 2, **dadurch gekennzeichnet, dass** der Tumor ein Tumor des zentralen Nervensystems ist.

4. In vitro-Verwendung eines Diagnosekits, das auf einem geeigneten Träger ein Mittel umfasst, das den Nachweis von nicht phosphoryliertem Vimentin erlaubt und/oder ein Mittel, das den Nachweis von phosphoryliertem Vimentin erlaubt, um die Aggressivität und/oder Invasivität eines Tumors zu bestimmen, wobei die signifikante Präsenz phosphorylierten Vimentins ein Indiz für die Nicht-Aggressivität und/oder Nicht-Invasivität des Tumors ist.

5. Verwendung nach Anspruch 4, **dadurch gekennzeichnet, dass** das Mittel zum Nachweis des nicht phosphorylierten Vimentins ausgewählt ist aus:
den Antikörpern, die mit dem ganzen oder einem Teil des Proteins reagieren, den Reagenzien, die keine Antikörper sind und in der Lage, auf spezifische Weise mit Vimentin zu interagieren, den Reagenzien der Modifizierung und/oder Spaltung des Proteins auf Ebene der Phosphorylierungsstellen, den radioaktiven Tracern.

6. Verwendung nach Anspruch 4, **dadurch gekennzeichnet, dass** das Mittel zum Nachweis des phosphorylierten Vimentins ausgewählt ist aus:
den Antikörpern, die mit dem ganzen oder einem Teil des Proteins reagieren, den Reagenzien, die keine Antikörper sind und in der Lage, auf spezifische Weise mit Vimentin zu interagieren, den Reagenzien der Modifizierung (kolorimetrische oder Fluoreszenzmarkierung) auf Ebene der Phosphorylierungsstellen, den Reagenzien und/oder Proteasen für die spezifische Spaltung des Proteins auf Ebene der Phosphorylierungsstellen, den radioaktiven Tracern.

7. Verwendung nach einem der Ansprüche 4 bis 6, **dadurch gekennzeichnet, dass** der Träger ausgewählt ist aus den Trägern chemischer Verbindungen, die in der Lage sind, mit phosphoryliertem oder nicht phosphoryliertem Vitimin zu interagieren, den Kunststoff-Dosierplatten mit mehreren Vertiefungen, den Polyacrylamidgelen oder anderen Matrices für die Separierung von Proteinen nach ihren physikalischchemischen Eigenschaften, den Latexkugeln oder anderen Materialien, die aktiviert sind, um spezifisch mit Vimentin zu interagieren.

8. Verfahren zur Identifizierung eines antikarzinogenen Mittels, das es ermöglicht, die Aggressivität und/oder die Invasivität eines Tumors durch Fördern der Phosphorylierung des Vimentins zu reduzieren, **dadurch gekennzeichnet, dass** es die Schritte umfasst:
- Kultivierung von Zellen, die nicht phosphoryliertes Vimentin produzieren,
- Herstellung des Kontakts der Zellen mit einem Testmittel,
- Analyse des produzierten Vimentins, um zu bestimmen, ob es nach Zellkultur im Kontakt mit dem Testmittel phosphoryliert ist oder nicht,
- Selektion des oder der Mittel, die in der Lage sind, die Phosphorylierung des Vimentins in den getesteten Krebszellen zu induzieren.

9. Verfahren nach Anspruch 8, **dadurch gekennzeichnet, dass** die Vimentin produzierenden Zellen Krebszellen sind.

10. Verfahren nach einem der Ansprüche 8 oder 9, **dadurch gekennzeichnet, dass** die Analyse des produzierten Vimentins gemäß dem Verfahren nach einem der Ansprüche 1 oder 2 durchgeführt wird.


### SEQUENCE LISTING

<110> GENOME EXPRESS INSERM
<120> VIMENTINE PHOSPHORYLEE COMME MARQUEUR DE L'AGRESSIVITE ET/OU L'INVASIVITE DES TUMEURS
<130> 66888 D22699
<140> EP05797071.7
   <141> 2005-09-15
<150> FR0409857
   <151> 2004-09-17
<160> 14
<170> PatentIn version 3.3
<210> 1
   <211> 9
   <212> PRT
   <213> Human
<400> 1
   Ile Ala Thr Tyr Arg Lys Leu Leu Glu
   1               5
<210> 2
   <211> 11
   <212> PRT
   <213> Human
<400> 2
   Ile Ala Phe Leu Lys Lys Leu His Glu Glu Glu
   1 5 10
<210> 3
   <211> 11
   <212> PRT
   <213> human
<400> 3
   Glu Ile Ala Phe Leu Lys Lys Leu His Glu Glu
   1 5 10
<210> 4
   <211> 14
   <212> PRT
   <213> human
<400> 4
   Ala Ala Asn Tyr Gln Asp Thr Ile Gly Arg Leu Gln Asp Glu
   1 5 10
<210> 5
   <211> 15
   <212> PRT
   <213> human
<400> 5
   Ala Ala Asn Arg Asn Asn Asp Ala Leu Arg Gln Ala Lys Gln Glu
   1 5 10 15
<210> 6
   <211> 14
   <212> PRT
   <213> human
<400> 6
   Ile Ala Phe Leu Lys Lys Leu His Glu Glu Glu Ile Gln Glu
   1 5 10
<210> 7
   <211> 17
   <212> PRT
   <213> human
<400> 7
   Ser Arg Ile Ser Leu Pro Leu Pro Asn Phe Ser Ser Leu Asn Leu Arg
   1               5                   10                  15
   Glu
<210> 8
   <211> 16
   <212> PRT
   <213> human
<400> 8
   Leu Asn Asp Arg Phe Ala Asn Tyr Ile Asp Lys Val Arg Phe Leu Glu
   1               5                   10                  15
<210> 9
   <211> 19
   <212> PRT
   <213> human
<400> 9
   Leu Glu Gln Leu Lys Gly Gln Gly Lys Ser Arg Leu Gly Asp Leu Tyr
   1               5                   10                  15
   Glu Glu Glu
<210> 10
   <211> 20
   <212> PRT
   <213> human
<400> 10
   Gly Glu Glu Ser Arg Ile Ser Leu Pro Leu Pro Asn Phe Ser Ser Leu
   1               5                   10                  15
   Asn Leu Arg Glu
               20
<210> 11
   <211> 18
   <212> PRT
   <213> human
<400> 11
   Arg Asp Asn Leu Ala Glu Asp Ile Met Arg Leu Arg Glu Lys Leu Gln
   1 5 10 15
   Glu Glu
<210> 12
   <211> 21
   <212> PRT
   <213> human
<400> 12
   Asn Thr Leu Gln Ser Phe Arg Gln Asp Val Asp Asn Ala Ser Leu Ala
   1 5 10 15
   Arg Leu Asp Leu Glu
   20
<210> 13
   <211> 23
   <212> PRT
   <213> human
<400> 13
   Ala Glu Asn Thr Leu Gln Ser Phe Arg Gln Asp Val Asp Asn Ala Ser
   1 5 10 15
   Leu Ala Arg Leu Asp Leu Glu
   20
<210> 14
   <211> 25
   <212> PRT
   <213> human
<400> 14
   Asn Thr Leu Gln Ser Phe Arg Gln Asp Val Asp Asn Ala Ser Leu Ala
   1 5 10 15
   Arg Leu Asp Leu Glu Arg Lys Val Glu
   20 25